# EUROPÄISCHE PATENTSCHRIFT

(11) **EP 1 090 652 B1**
(45) Veröffentlichungstag und Bekanntmachung des Hinweises auf die Patenterteilung: **24.03.2004**
(21) Anmeldenummer: 00120899.0
(22) Anmeldetag: 26.09.2000
(51) Int. Cl.: A61M 5/32

(54) **Spritzenkörper mit Nadelschutz**
Syringe body with needle shield
Corps de seringue avec protecteur d'aiguille

(30) Priorität: 06.10.1999 DE 19947998
(43) Veröffentlichungstag der Anmeldung: 11.04.2001
(73) Patentinhaber: Bünder Glas GmbH, 32257 Bünde (DE)
(72) Erfinder: Geiger, Andreas, 32278 Kirchlengern (DE); Stohlmann, Erhard, 32257 Bünde (DE); Theiling, Manfred, 32257 Bünde (DE)
(74) Vertreter: Schirmer, Siegfried, Dipl.-Ing.

(56) Entgegenhaltungen:
- FR-A- 2 770 405
- US-A- 5 201 720
- US-A- 5 713 871
- US-A- 5 891 092

## Beschreibung

Die Erfindung betrifft einen Spritzenkörper mit einem, auf dem Spritzenkörper zwischen einer zurückgezogenen Ausgangsstellung und einer vorgeschobenen, eine am Spritzenkörper angebrachte Nadel abdeckenden Shutzstellung in Längsrichtung verschiebbaren Nadelschutzes, wobei an einem der Nadel entgegengesetzten Ende des Spritzenkörpers eine Griffplatte angeformt ist und in dem Spritzenkörper eine Kolbenstange zwischen einer Ausgangsstellung und einer Injektionsstellung längsverschieblich geführt ist.

Aus der EP 0 734 738 A2 ist eine Sicherheitsspritze mit einer Schutzhülse bekannt. Bei dieser bekannten Spritze ist die Schutzhülse in der zurückgezogenen Stellung durch Vorsprünge festgehalten, die an einem Kragen anstehen. Erst wenn die Schutzhülse auf dem Spritzenzylinder so verdreht wird, daß die Vorsprünge mit Ausnehmungen in dem Kragen fluchten, läßt sich die Schutzhülse zur Nadelspitze hin verschieben. In der Schutzstellung wird die Schutzhülse durch Rastzähne festgehalten, die mit einer Rastwand des Kragens in Eingriff kommen.

Aus der FR-A-2770405 ist ein Spritzenkörper mit Nadelschutz nach dem Oberbegriff des Anspruchs 1 bekannt, bei dem die Kolbenstange mit Klauen versehen ist, die in eine Nut des Nadelschutzes eingreifen, so daß die Kolbenstange und der Nadelschutz zunächst während des Injektionsvorgangs, d.h. während der Bewegung der Kolbenstange von der Ausgangsstellung in die Injektionsstellung, gemeinsam bewegt werden, wobei sich ein vorderes Ende des Nadelschutzes entlang des Spritzenkörpers vorschiebt. Mit Erreichen der Injektionsstellung lösen sich die Klauen der Kolbenstange aus der Nut des Nadelschutzes, so daß dieser unabhängig von der Kolbenstange in eine vorgeschobene Schutzstellung bewegt werden kann. Bei diesem Stand der Technik ist als nachteilig anzusehen, daß sich der Nadelschutz bereits während des Injektionsvorgangs entlang des Spritzenkörpers vorschiebt, was bei einer Injektion sehr störend sein kann.

Der Erfindung liegt die Aufgabe zugrunde, einen gattungsgemäßen Nadelschutz unter Vermeidung des vorstehend genannten Nachteils auszubilden.

Diese Aufgabe wird erfindungsgemäß durch die Merkmale des Anspruchs 1 gelöst.
Vorteilhafte Ausgestaltungen sind in den Unteransprüchen aufgezeigt.

Ein Ausführungsbeispiel der Erfindung ist in der Zeichnung dargestellt und wird nachfolgend näher beschrieben. Es zeigen jeweils in einem Längsschnitt:

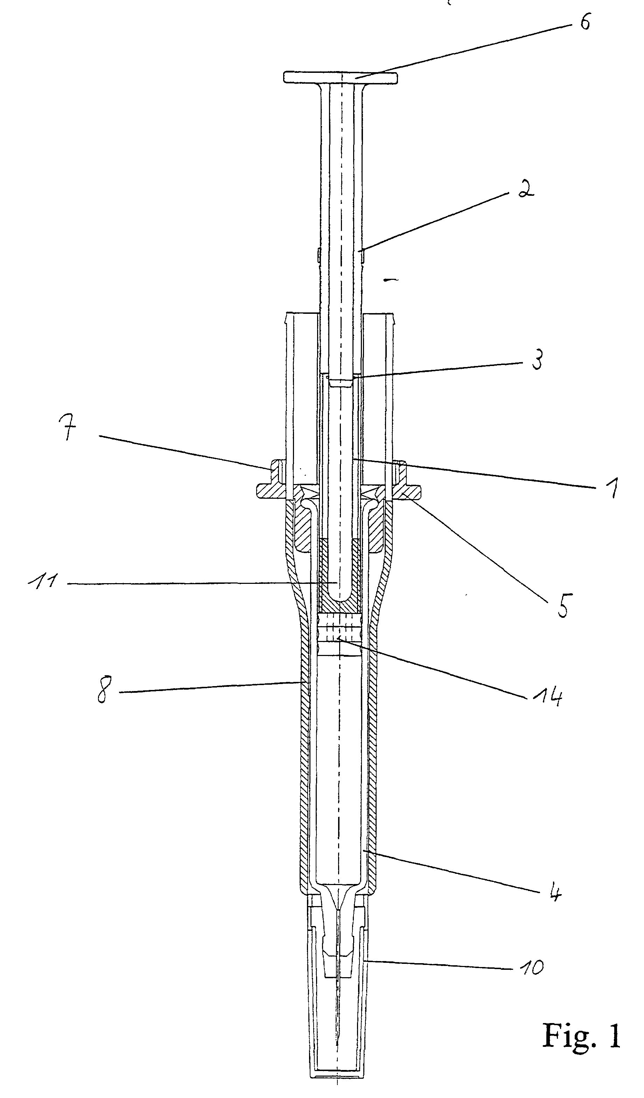

- Fig. 1: einen Spritzenkörper mit bekannter Schutzkappe und mit einem verschiebbaren Nadelschutz in Ausgangsstellung der Kolbenstange;
- Fig. 2: einen Spritzenkörper mit verschobener Kolbenstange;
- Fig. 3: einen Spritzenkörper mit teleskopartig ineinander geschobener Kolbenstange und verschobenem Nadelschutz in Funktionsstellung und

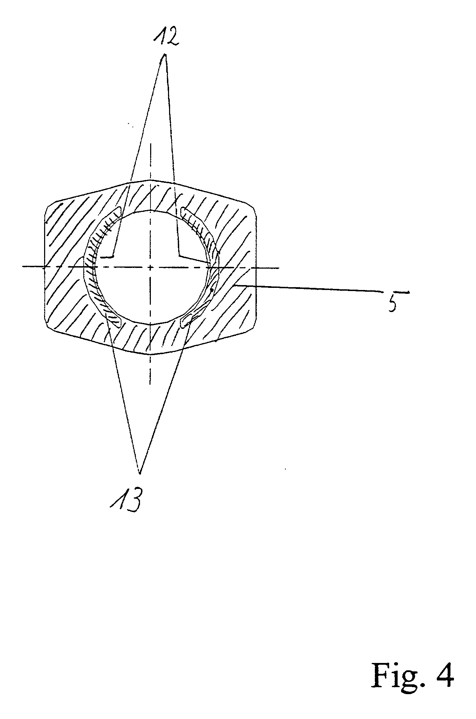

- Fig. 4: einen Schnitt nach Linie A - B der Fig. 1.

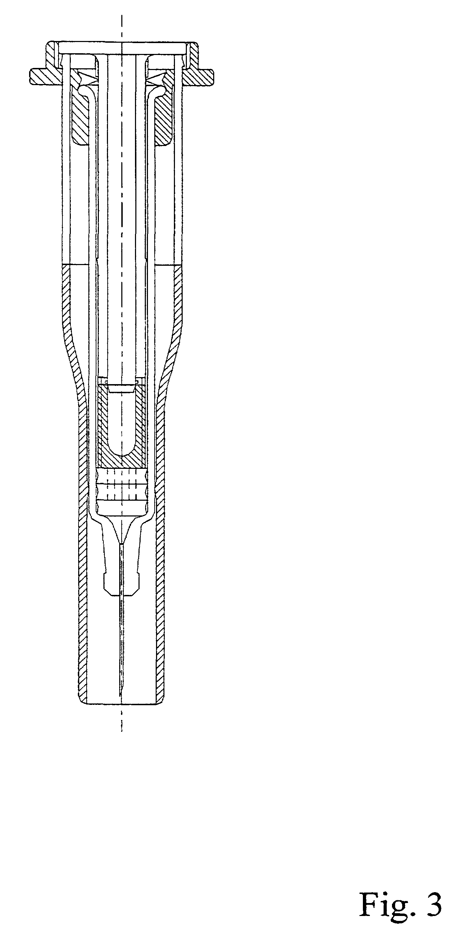

Die Kolbenstange 1; 2 ist zweigeteilt ausgebildet, deren Teile durch eine mit einer Sollbruchstelle versehenen Sperrhaltung 3 verbunden sind. Die Kolbenstange besitzt einen Aufnahmeteil 1, in das ein einen kleineren Durchmesser aufweisendes Teil 2 teleskopartig einschiebbar ist. Das einschiebbare Teil 2 der Kolbenstange besitzt am freien Ende eine Kolbenstangengriffplatte 6. Die Griffplatte 5 des Spritzenkörpers 4 weist eine Kreisringaufnahme 7 auf, die senkrecht zur Außenseite der Griffplatte 5 verläuft und deren Durchmesser größer ist als der Durchmesser der Kolbenstangengriffplatte 6. Wie aus Fig. 3 ersichtlich, liegt die Kolbenstangengriffplatte 6 im eingeschobenen Zustand der Kolbenstange 1; 2 bündig mit der Stirnseite der Kreisringaufnahme 7 der Griffplatte 5 des Spritzenkörpers 4.

Der Aufnahmeteil 1 der Kolbenstange besitzt zwei längsverlaufende Rastnuten und der einschiebbare Teil 2 der Kolbenstange zwei längsverlaufende Rippen, die äquivalent zu den längsverlaufenden Rastnuten ausgebildet sind.

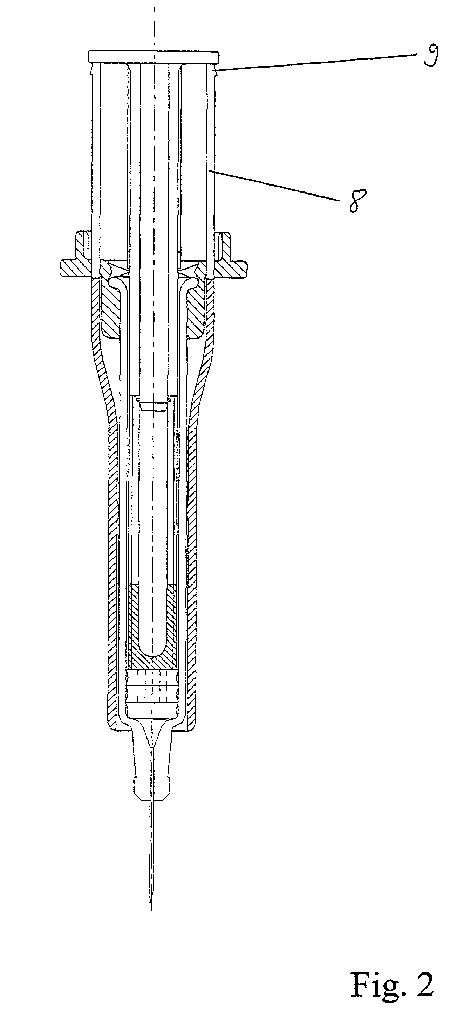

Die Innenseite der Kolbenstangengriffplatte 6 kommt auf der Stirnseite des Nadelschutzes 8 zur Anlage, vgl. Fig. 2. In dieser Position liegt der Kolbenstangengummi am Ende des Spritzenkörpers 4 an, d. h. die Injektion ist beendet. Bis zu diesem Vorgang wirkt der zweiteilige Kolben 1; 2 als Einheit. Bei weiterem Druck auf die Kolbenstangengriffplatte 6 wird die Sperrhalterung 3 zwischen den beiden Teilen der Kolbenstange 1; 2 zerbrochen und die Kolbenstange teleskopartig ineinander gefahren. Da die Kolbenstangengriffplatte 6 auf der Stirnseite des Nadelschutzes 8 aufliegt, wird bei weiterem Einschieben der Kolbenstange 2 der Nadelschutz 8 mitgenommen und deckt so in einem einzigen Arbeitsgang die eingeklebte Nadel 15 ab. Diese Position ist in Fig. 3 dargestellt. Der Verschiebevorgang des Nadelschutzes 8 wird durch einen umlaufenden Widerhaken 9 begrenzt. Dieser Widerhaken 9 liegt im verschobenen Zustand des Nadelschutzes 8 an der Griffplatte 5 des Spritzenkörpers 4 an.

Der Nadelschutz 8 weist zwei längsverlaufende und gegenüberliegende Aussparungen 11 und die Griffplatte 5 des Spritzenkörpers 4 zwei bogenförmige Aussparungen 12 auf. Der Nadelschutz 8 ist mit den zwischen den Aussparungen 11 liegenden Bereichen 13 in den bogenförmigen Aussparungen 12 zwangsgeführt. Die bekannte Nadelschutzkappe ist mit 10 bezeichnet.

### Aufstellung der Bezugszeichen:

- 1: Kolbenstange (Aufnahmeteil)
- 2: Kolbenstange (einschiebbarer Teil)
- 3: Sperrhalterung
- 4: Spritzenkörper
- 5: Griffplatte von 4
- 6: Kolbenstangengriffplatte
- 7: Kreisringaufnahme von 5
- 8: Nadelschutz
- 9: Widerhaken
- 10: Nadelschutzkappe
- 11: Aussparungen in 8
- 12: bogenförmige Aussparungen in 5
- 13: Bereich zwischen 11
- 14: Kolbenstangengummi
- 15: Nadel

## Patentansprüche

1. Spritzenkörper (4) mit einem auf dem Spritzenkörper (4) zwischen einer zurückgezogenen Ausgangsstellung und einer vorgeschobenen, eine am Spritzenkörper angebrachte Nadel abdeckenden Schutzstellung in Längsrichtung verschiebbaren Nadelschutz (8), wobei an einem der Nadel entgegengesetzten Ende des Spritzenkörpers (4) eine Griffplatte (5) angeformt ist und in dem Spritzenkörper (4) eine Kolbenstange (1, 2) zwischen einer Ausgangsstellung und einer vollständig eingeschobenen Injektionsstellung längsverschieblich geführt ist, **dadurch gekennzeichnet, daß** die Kolbenstange (1, 2) zweiteilig, teleskopartig ineinanderschiebbar ausgebildet, zwischen Ausgangs- und Injektionsstellung unabhängig von dem Nadelschutz (8) bewegbar und anschließend an die Injektionsstellung nach Überwinden einer zwischen den beiden ineinanderschiebbaren Teilen (1, 2) der Kolbenstange vorhandenen Sperrhalterung (3) in eine ineinandergeschobene Stellung bringbar ist, wobei eine am freien Ende des einschiebbaren Teils (2) der Kolbenstange vorgesehene Kolbenstangengriffplatte (6) beim Ineinanderschieben der Kolbenstange (1, 2) mit dem Nadelschutz (8) zusammenwirkt und diesen in die Schutzstellung bringt.

2. Spritzenkörper nach Anspruch 1, **dadurch gekennzeichnet, daß** die Sperrhalterung (3) eine Sollbruchstelle aufweist.

3. Spritzenkörper nach Anspruch 1 oder 2, **dadurch gekennzeichnet, daß** die Griffplatte (5) des Spritzenkörpers (4) mit einer Kreisringaufnahme (7) versehen ist.

4. Spritzenkörper nach Anspruch 3, **dadurch gekennzeichnet, daß** der Durchmesser der Kreisringaufnahme (7) größer ist als der Durchmesser der Kolbenstangengriffplatte (6).

5. Spritzenkörper nach Anspruch 4, **dadurch gekennzeichnet, daß** die Kolbenstangengriffplatte (6) im ineinandergeschobenen Zustand der Kolbenstange (1; 2) bündig liegt mit der Stirnseite der Kreisringaufnahme (7).

6. Spritzenkörper nach einem der Ansprüche 2 bis 5, **dadurch gekennzeichnet, daß** der Aufnahmeteil (1) der Kolbenstange (1,2) mit mindestens einer längsverschieblichen Rastnut versehen ist.

7. Spritzenkörper nach einem der Ansprüche 2 bis 6, **dadurch gekennzeichnet, daß** der einschiebbare Teil (2) der Kolbenstange mit mindestens einer längsverlaufenden Rippe versehen ist.

8. Spritzenkörper nach Anspruch 6 und 7, **dadurch gekennzeichnet, daß** die mindestens eine längsverlaufende Rippe des einschiebbaren Teils (2) der Kolbenstange äquivalent zu den längsverlaufenden Rastnuten des Aufnahmeteils (1) der Kolbenstange (1, 2) ausgebildet ist.

9. Spritzenkörper nach einem der Ansprüche 1 bis 8, **dadurch gekennzeichnet, daß** eine Innenseite der Kolbenstangengriffplatte (6) auf einer Stirnseite des Nadelschutzes (8) zur Anlage kommt.

10. Spritzenkörper nach einem der Ansprüche 1 bis 9, **dadurch gekennzeichnet, daß** der Nadelschutz (8) mit einem Widerhaken (9) versehen ist.

11. Spritzenkörper nah Anspruch 10, **dadurch gekennzeichnet, daß** der Widerhaken (9) in der Schutzstellung des Nadelschutzes (8) an der Griffplatte (5) des Spritzenkörpers (4) anliegt.

12. Spritzenkörper nach Anspruch 10 oder 11, **dadurch gekennzeichnet, daß** der Widerhaken (9) umlaufend angeordnet ist.

13. Spritzenkörper nach einem der Ansprüche 1 bis 12, **dadurch gekennzeichnet, daß** der Nadelschutz (8) zwei längsverlaufende und gegenüberliegende Aussparungen (11) aufweist.

14. Spritzenkörper nach einem der Ansprüche 1 bis 13, **dadurch gekennzeichnet, daß** die Griffplatte (5) des Spritzenkörpers (4) zwei bogenförmige Aussparungen (12) aufweist.

15. Spritzenkörper nach Anspruch 13 und 14, **dadurch gekennzeichnet, daß** der Nadelschutz (8) mit zwischen den Aussparungen (11) liegenden Bereichen (13) in den bogenförmigen Aussparungen (12) zwangsgeführt ist.

## Claims

1. Syringe body (4) with a needle protector (8) displaceable in longitudinal direction between a retracted initial setting and an advanced protective setting covering a needle mounted at the syringe body, wherein a grip plate (5) is integrally formed at an end of the syringe body (4) opposite the needle and a piston rod (1, 2) is guided in the syringe body (4) to be longitudinally displaceable between an initial setting and a fully withdrawn injection setting, **characterised in that** the piston rod (1, 2) is constructed in two parts to be telescopically pushable together and movable between initial setting and injection setting independently of the needle protector (8) and can subsequently be brought at the injection setting into a pushed-together setting after overcoming a locking retainer (3) present between the two parts (1, 2), which can be pushed into one another, of the piston rod, wherein a piston rod grip plate (6) provided at the free end of the part (2), which can be pushed in, of the piston rod co-operates with the needle protector (8) when the piston rod (1, 2) is pushed together and brings this protector into the protective setting.

2. Syringe body according to claim 1, **characterised in that** the locking retainer (3) has a frangible location.

3. Syringe body according to one of claims 1 and 2, **characterised in that** the grip plate (5) of the syringe body (4) is provided with a circularly annular mount (7).

4. Syringe body according to claim 4, **characterised in that** the diameter of the circularly annular mount (7) is greater than the diameter of the piston rod grip plate (6).

5. Syringe body according to claim 4, **characterised in that** the piston rod grip plate (6) in the pushed-together state of the piston rod (1; 2) is flush with the end face of the circularly annular mount (7).

6. Syringe body according to one of claims 2 to 5, **characterised in that** the receiving part (1) of the piston rod (1, 2) is provided with at least one longitudinally displaceable detent groove.

7. Syringe body according to one of claims 2 to 6, **characterised in that** the part (2), which can be pushed in, of the piston rod is provided with at least one longitudinally extending rib.

8. Syringe body according to claim 6 and 7, **characterised in that** the at least one longitudinally extending rib of the part (2), which can be pushed in, of the piston rod is constructed to be equivalent to the longitudinally extending detent grooves of the receiving part (1) of the piston rod (1, 2).

9. Syringe body according to one of claims 1 to 8, **characterised in that** an inner side of the piston rod grip plate (6) comes into contact with an end face of the needle protector (8).

10. Syringe body according to one of claims 1 to 9, **characterised in that** the needle protector (9) is provided with a barb (9).

11. Syringe body according to claim 10, **characterised in that** the barb (9) in the protective setting of the needle protector (8) bears against the grip plate (5) of the syringe body (4).

12. Syringe body according to claim 10 or 11, **characterised in that** the barb (9) is arranged to be encircling.

13. Syringe body according to one of claims 1 to 12, **characterised in that** the needle protector (8) has two longitudinally extending and mutually opposite recesses (11).

14. Syringe body according to one of claims 1 to 13, **characterised in that** the grip plate (5) of the syringe body (4) has two curvilinear recesses (12).

15. Syringe body according to claim 13 and 14, **characterised in that** the needle protector (8) is constrainedly guided in the curvilinear recesses (12) by regions (13) lying between the recesses (11).

## Revendications

1. Corps de seringue (4) muni d'un protecteur d'aiguille (8) qu'on peut faire coulisser sur le corps de seringue (4) dans la direction longitudinale entre une position de départ rétractée en arrière et une position de protection avancée, qui recouvre une aiguille fixée au corps de seringue, dans lequel un repose-doigts (5) est formé à une extrémité du corps de seringue (4) qui est à l'opposé de l'aiguille, et une tige de piston (1, 2) est guidée en translation longitudinale dans le corps de seringue (4) entre une position de départ et une position d'injection entièrement enfoncée,
**caractérisé en ce que**
la tige de piston (1, 2) est réalisée en deux parties qui peuvent s'emmancher télescopiquement l'une dans l'autre, peut se déplacer indépendamment du protecteur d'aiguille (8) entre la position de départ et la position d'injection et, ensuite, peut être amenée à la position d'injection après avoir surmonté une retenue (3) présente entre les deux parties (1, 2) de la tige de piston qui peuvent s'emmancher l'une dans l'autre, pour être placée dans une position d'emmanchement télescopique, alors qu'un repose-doigts de tige de piston (6) prévu à l'extrémité libre de la partie rentrante (2) de la tige de piston, coopère avec le protecteur d'aiguille (8) avec la tige de piston (1, 2) en état d'emmanchement télescopique, et place ce dernier dans la position de protection.

2. Corps de seringue selon la revendication 1,
**caractérisé en ce que**
la retenue (3) présente une zone d'amorce de rupture.

3. Corps de seringue selon une des revendications 1 à 2,
**caractérisé en ce que**
le repose-doigts (5) du corps de seringue (4) est muni d'un logement en couronne de cercle (7).

4. Corps de seringue selon la revendication 3,
**caractérisé en ce que**
le diamètre du logement en couronne de cercle (7) est plus grand que le diamètre du repose-doigts de tige de piston (6).

5. Corps de seringue selon la revendication 4,
**caractérisé en ce que**
le repose-doigts de tige de piston (6) se trouve de niveau avec le côté frontal du logement en couronne de cercle (7) dans l'état emmanché télescopiquement de la tige de piston (1, 2).

6. Corps de seringue selon une des revendications 2 à 5,
**caractérisé en ce que**
la partie réceptrice (1) de la tige de piston (1, 2) est munie d'au moins une rainure d'enclenchement mobile en translation longitudinale.

7. Corps de seringue selon une des revendications 2 à 6,
**caractérisé en ce que**
la partie rentrante (2) de la tige de piston est munie d'au moins une nervure longitudinale.

8. Corps de seringue selon la revendication 6 et 7,
**caractérisé en ce que**
l'au moins une nervure longitudinale de la partie rentrante (2) de la tige de piston est de forme équivalente à celle des rainures d'enclenchement longitudinales de la partie réceptrice (1) de la tige de piston (1, 2).

9. Corps de seringue selon une des revendications 1 à 8,
**caractérisé en ce qu'**
un côté intérieur du repose-doigts de tige de piston (6) entre en contact avec un côté frontal du protecteur d'aiguille (8).

10. Corps de seringue selon une des revendications 1 à 7,
**caractérisé en ce que**
le protecteur d'aiguille (8) est muni d'une barbe (9).

11. Corps de seringue selon la revendication 10,
**caractérisé en ce que**
la barbe (9) est en appui contre le repose-doigts (5) du corps de seringue (4) dans la position de protection du protecteur d'aiguille (8).

12. Corps de seringue selon la revendication 10 ou 11,
**caractérisé en ce que**
la barbe (9) est disposée circonférenciellement.

13. Corps de seringue selon une des revendications 1 à 12,
**caractérisé en ce que**
le protecteur d'aiguille (8) présente deux évidements longitudinaux et opposés (11).

14. Corps de seringue selon une des revendications 1 à 13,
**caractérisé en ce que**
le repose-doigts (5) du corps de seringue (4) présente deux évidements (12) en forme d'arc.

15. Corps de seringue selon la revendication 13 et 14,
**caractérisé en ce que**
le protecteur d'aiguille (8) est guidé positivement dans les évidements en forme d'arc (12) par des régions (13) situées entre les évidements (11).
